# EUROPEAN PATENT SPECIFICATION

(11) **EP 2 936 943 B1**
(45) Date of publication and mention of the grant of the patent: **14.06.2017**
(21) Application number: 13815796.1
(22) Date of filing: 16.12.2013
(51) Int. Cl.: H05H 1/24, A61B 18/04, A61L 2/14, H01J 37/32

(54) **DEVICE FOR PROVIDING A FLOW OF PLASMA**
VORRICHTUNG ZUR BEREITSTELLUNG EINES PLASMASTROMS
DISPOSITIF PERMETTANT DE PRODUIRE UN FLUX PLASMATIQUE

(30) Priority: 18.12.2012 GB 201222840
(43) Date of publication of application: 28.10.2015
(73) Proprietor: Linde Aktiengesellschaft, 80331 München (DE)
(72) Inventor: HOLBECHE, Thomas Bickford, Guildford GU2 7XY (GB)
(74) Representative: Christie, Gemma Louise
(86) International application number: PCT/GB2013/000550
(87) International publication number: WO 2014/096755

(56) References cited:
- WO-A1-2009/146432
- WO-A1-2011/092186
- WO-A1-2012/005132
- US-A1- 2012 064 016
- JUSTIN ZITO ET AL: "Microscale Dielectric Barrier Discharge Plasma Actuators: Performance Characterization and Numerical Comparison", 43RD AIAA PLASMADYNAMICS AND LASERS CONFERENCE, 25 June 2012 (2012-06-25), XP055105652, Reston, Virigina DOI: 10.2514/6.2012-3091 ISBN: 978-1-62-410187-8

## Description

### FIELD OF THE INVENTION

The present description relates to a device for providing a flow of atmospheric plasma. The invention is set out in the appended claims.

### BACKGROUND OF THE INVENTION

Systems for the generation of non-thermal gas plasma are known and have utility in a number of fields such as industrial, dental, medical, cosmetic and veterinary fields for the treatment of the human or animal body. Non-thermal gas plasma generation can be employed to promote coagulation of blood, cleaning, sterilisation and removal of contaminants from a surface, disinfection, reconnection of tissue and treatment of tissue disorders without causing significant thermal tissue damage. In order to be tolerable for a patient, the atmospheric plasma flow, including ions and non-ionised gas, should be maintained at an acceptable temperature, preferably below about 40°C.

In such plasma devices, it is additionally desirable to conserve power and to increase the amount of active species (e.g. OH radicals) in the plasma which is delivered to the treatment region whilst also conserving gas consumption. WO-A-2012/005132 describes a plasma treatment equipment comprising a plasma cell for generating plasma and a high-frequency electric field shield member.

### SUMMARY OF THE INVENTION

The present description provides a device for forming at an ambient atmospheric pressure a gaseous plasma comprising active species for treatment of a treatment region, the device comprising: at least one plasma cell for forming said gaseous plasma for treating the treatment region, the at least one plasma cell comprising: an inlet for receiving gas from a source and an outlet for discharging active species generated in the cell; a dielectric substrate enclosed around a flow path for gas conveyed from the inlet to the outlet; an electrode formed on or in the dielectric substrate for energising gas along the flow path to form active species; and a protective coating made of a dielectric formed on an inner surface of the dielectric substrate for protecting the dielectric substrate from reaction with the active species, the device further comprising: a nozzle outlet for discharging said gaseous plasma; an earth electrode comprising a dielectric substrate and an electrode formed on or in the dielectric substrate, wherein the earth electrode substantially surrounds and at least partially overlaps the at least one plasma cell, wherein the outermost end of the earth electrode is closer to the nozzle outlet than the outlet of the plasma cell.

The device of the present description is advantageous as the interaction of the fields produced by the at least one plasma cell and the earth electrode serves to reduce the power required to supply the non-thermal plasma.

The dielectric substrate of the at least one plasma cell and/or the earth electrode is made of a polyimide. Polyimides have the advantage that they are lightweight, flexible, resistant to heat and chemicals, have a high dielectric strength and are able to act as a substrate for printed electrical components.

Preferably, the protective coating is made of a material selected from one of PTFE, FEP or silicone rubber being generally un-reactive with the active species. The protective coating may be made of a material which is generally unreactive with the active species generated in the cell.

In one preferred embodiment the electrode of the at least one plasma cell and/or the earth electrode is formed by patterning an electrically conductive material on the respective dielectric substrate. The electrode may preferably be printed, or may be formed of a fibrous matrix transferred onto the respective dielectric substrate.

Preferably, the dielectric substrate of the at least one plasma cell is flexible and is shaped to define the flow path. In a preferred embodiment the dielectric substrate of the at least one plasma cell is formed by a flexible tube enclosing the flow path.

A protective sheath is preferably formed around the dielectric substrate and electrode of the at least one plasma cell to protect the electrode.

In one preferred embodiment the device comprises a plasma cell array having a plurality of plasma cells.

In another aspect, the present description relates to a plasma cell for a device as herein described. A device according to the description may be made by forming an electrode onto a dielectric substrate made of a polyimide, configuring the dielectric substrate to form a flow path for gas from a cell inlet to a cell outlet and forming a protective dielectric coating on an inner surface of the dielectric substrate for protecting the substrate from reaction with the active species.

The electrode may be patterned onto the dielectric substrate.

The patterned electrode may be deposited on the dielectric substrate by printing or formed of a fibrous matrix transferred onto the dielectric substrate.

The dielectric substrate is flexible and following formation of the electrode on the substrate the substrate is shaped to enclose the flow path between the inlet and the outlet.

The dielectric substrate may be shaped to correspond with the shape of a former inside the device.

The protective coating may be made of a material which is generally unreactive with the active species generated in the cell.

The method may comprise forming a protective sheath made of a dielectric around the dielectric substrate and patterned electrode. The present invention is set out in the appended claims. The embodiments, aspects or examples according to the present description that do not fall within the scope of said claims are provided for illustrative purposes only and do not form part of the present invention.

### BRIEF DESCRIPTION OF THE DRAWINGS

In order that the description may be more clearly understood, several embodiments thereof, which are given by way of example only, will now be described in more detail with reference to the accompanying drawings, in which:
Figure 1 shows a device for forming a plasma;
Figure 2 shows a plasma cell of the device in more detail;
Figure 3 shows in Figure 3a a plasma cell in perspective, in Figure 3b the plasma cell in longitudinal section, in Figure 3c in lateral section, and in Figure 3d the electrodes of the cell;
Figure 4 shows in Figure 4a a plasma cell in perspective, in Figure 4b the plasma cell in longitudinal section, in Figure 4c in lateral section, and in Figure 4 the plasma cell in plan;
Figure 5 shows a plasma cell in partial cut-away;
Figure 6 shows a device having a plasma cell array;
Figure 7 shows a device having an alternative arrangement; and
Figure 8 shows a detailed view of the nozzle portion of Figure 7.

### DETAILED DESCRIPTION OF ILLUSTRATED EMBODIMENTS

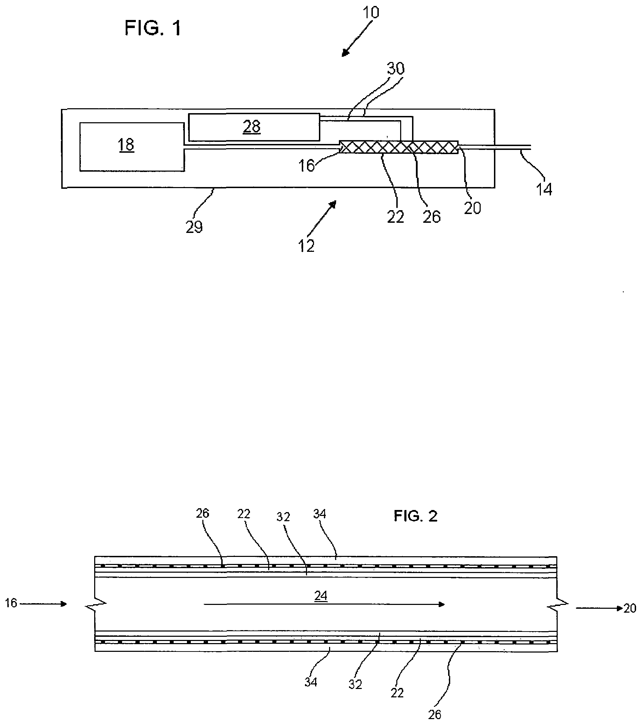

Referring to Figure 1, there is shown a device 10 for providing a flow of plasma for treatment of a treatment region, which may be part of a human or animal body such as teeth. The device comprises a plasma cell 12 for forming at an ambient atmospheric pressure a gaseous plasma comprising active species to be discharged through nozzle 14 for treating the treatment region. The pressure need not be controlled to maintain strict ambient atmospheric pressure but significant positive or negative pressure should generally be avoided in the example of Figure 1.

The plasma cell 12 comprises an inlet 16 for receiving gas from a source 18 and an outlet 20 for discharging active species generated in the cell. A dielectric substrate 22 is enclosed around a flow path 24 for gas conveyed from the inlet to the outlet. An electrode 26 is formed on an outer surface of the dielectric substrate and connected to a source of electrical power 28 by electrical connectors 30 for energising gas along the flow path to form active species. The electrode 26 may be embedded in the dielectric substrate 22 or sandwiched between substrates. The source of electrical power is designed to drive the electrodes with a suitably high voltage and frequency to energise gas in the cell, for example 2.5 kV RMS at 100 MHz, however the voltage must not exceed the dielectric strength of the dielectric substrate to avoid conductive pathways being formed through the substrate. The source should also be configured not to overload the electrode configuration causing melting and consequent short circuiting of tracts of a patterned electrode configuration. A housing 29 houses the components of the device.

An enlarged section II taken through the plasma cell is shown in Figure 2. The electrode 26 in this example takes the form of a spiral and is transferred onto the outer surface of the generally cylindrical dielectric substrate 22. The electrode has a regular pattern to produce a generally uniform electric field in the plasma cell. A protective lining 32 is located on an inner surface of the dielectric substrate for resisting reaction of the active species generated in the cell 12 with the dielectric substrate 22. Such reaction if allowed would degrade the dielectric substrate and reduce its electrically insulating properties, or dielectric strength, and result in electrical conduction between the electrode and the gas in the cell. Such conduction may lead to arcing which heats the plasma, drains power and can produce undesirable active species. A protective sheath 34 surrounds the electrode and the dielectric substrate and protects the inner cell components from physical damage. The sheath 34 in this example is made of a dielectric which protects the region external to the plasma cell from exposure to high voltage. The region external to the plasma cell typically contains air, and the high voltage would, if not protected by the sheath, produce ozone by energising oxygen in the air.

The protection provided by the protective lining 32 means that the choice of materials for the dielectric substrate 22 is larger than would be the case in the absence of the protective lining 32. In the latter case, the substrate 22 would be required to be unreactive with the active species generated in the cell in addition to its required electrical properties. The active species are dependent upon the source gas from which the plasma is generated and may be argon or nitrogen. Accordingly, the substrate 22 may be made of polyimide which has suitable electrical properties but is generally reactive with active species. The protective lining 32 may be made of a material such as PTFE, FEP or silicone rubber being generally un-reactive with the active species. The composite structure of the cell provides an arrangement which has the required electrical properties but will not significantly degrade during use.

The dielectric substrate 22 may be made of any suitable dielectric medium and is preferably thin having a thickness of less than 5 mm, preferably less than 2 mm and more preferably less than 1 mm. Since the electric field generated across the discharge gas in the cell is reduced by increasing thickness, a thin substrate allows a higher strength field to be generated with reduced power consumption. However, it will be noted that many dielectric mediums have insufficient strength particularly when thin to resist breaking down when exposed to an electric field which is sufficiently high to generate an atmospheric plasma in the chamber. Accordingly, the dielectric strength of the selected dielectric substrate 22 should be sufficient to resist significant electrical conduction from the electrode to the gas in the cell. The dielectric material may be polyimide which has good electrical properties and is a flexible material meaning that it can be configured into any one of a number of different shapes, as will be described in more detail below.

Polyimides are polymers of imide monomers. Polyimides are lightweight, flexible, resistant to heat and chemicals, have a high dielectric strength and are able to act as a substrate for printed electrical components. Suitable polyimides for use in the invention and their preparation are described in, for example, US 3 179 634. A well known procedure for preparing polyimides is the two step poly(amic acid) process which involves reacting a dianhydride and a diamine at ambient conditions in a dipolar aprotic solvent such as N,N-dimethylacetamide (DMAc) or N-methylpyrrolidone (NMP) to yield the corresponding poly(amic) acid. This acid is then cyclised into the final polyimide. Such polyimides are sold commercially, notably under the trade mark KAPTON. The polyimide used most extensively in KAPTON products is believed to utilise the monomer pyromellitic dianhydride and 4,4' - oxydianiline.

Some commercial polyimide products are laminates with other plastics materials. Such laminates are disclosed in US 3 616 177 and US 2005/0013988 A1. The latter document specifically relates to dielectric substrates comprising a polyimide core layer and a high temperature fluoropolymer bonding layer.

It is also known to compound a polyimide with graphite or glass fibre so as to enhance its flexural strength and with metal so as to enhance its thermal conductivity. It is further known to provide grades of polyimide that are resistant to electrical corona discharge. For example, such products are commercially available as KAPTON CR and KAPTON FCR. Corona discharge-resistant forms of polyimide are known from, for example, US 3 389 111. The compositions disclosed therein contain certain organo-metallic compounds, particularly aromatic, aliphatic or araliphatic compounds of elements selected from Groups IVb and Vb of the Periodic Table of elements and iron, in which the metal is bonded through carbon to the organic portion of the molecule.

Another suitable polyimide is APICAL polyimide film which is an AF type aromatic polyimide made by Kaneka Texas Corporation. This polyimide has a dielectric strength in a range of 118 to 197 kV/mm depending on the particular film selected.

The electrodes 26 may be made from copper and printed onto the dielectric substrate 22 by techniques used in the fabrication of printed circuit boards, such as deposition or etching. However, the electrode pattern is configured to generate a high electric field in the plasma cell, whereas in PCBs, a high electric field is generally undesirable. Further in PCBs, the wiring is formed on one side of a substrate and acts as electrical conductors predominantly used for carrying electrical signals between components located on the other side of the substrate by interconnecting vias. In the present invention, the electrode pattern does not carry signals and is designed for use with high electrical potentials of for example 1 kV (or much greater).

The protective sheath 34 constitutes a physical barrier between the electrode pattern and substrate on the one hand and ambient conditions in the device and also provides structural support maintaining the cell in a generally cylindrical or other desired configuration. Accordingly, the protective sheath 34 may be made of a thermoplastic such as polyether block amide. The protective sheath 34 is also preferably a dielectric providing an electrical insulation between the electrode and the exterior of the plasma cell. Alternatively, a dielectric layer may overlay the dielectric substrate 22 and electrode 26 and one or more other layers may overlay the dielectric layer 22.

Additional layers may be provided in the laminated plasma cell, such as one or more adhesive layers, one or more additional electrode patterns, or one or more dielectric layers.

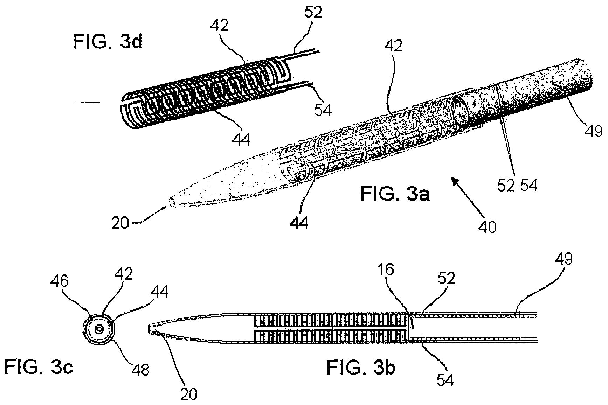

Referring to Figure 3, a plasma cell 40 is shown in more detail in which the or each electrode is transferred to the dielectric substrate by printing, such as by deposition or etching. Like reference numerals will be used to denote like features discussed above and will not be explained again for brevity. The cell 40 comprises first electrode 42 and second electrode 44 both printed on a dielectric substrate 46 by printing techniques known in the fabrication of PCBs. A second dielectric layer 48 covers the patterned electrodes and protects and electrically insulates the cell. A gas conduit 49 conveys gas from a source of gas to the plasma cell. A protective lining 32 is not shown in Figure 3 for simplicity of the drawings.

In a preferred method of manufacture of the plasma cell, the electrode(s) 42, 44 are printed on a generally planar dielectric substrate such as polyimide which is flexible so that after printing the substrate can be formed into a desired configuration, which in this example is a cylinder with a tapering front portion forming the cell outlet 20. The generally rectangular planar substrate is formed into a cylinder and then longitudinal sides of the substrate are joined and fixed to secure the substrate in a cylindrical configuration. In this regard, printing of the electrode(s) on a planar substrate is more readily and inexpensively achieved than by printing on a cylindrical substrate and standard PCB manufacturing equipment is available for printing on planar substrates. Of course though, the present invention does not preclude printing or otherwise patterning the electrode on a cylindrical substrate.

Flexible electronic circuits, or so-called flex circuits, are known in other technical fields and are used in for example cameras and cell phones. In such fields electronic components are mounted on flexible plastic substrates, such as polyimide, PEEK or transparent conductive polyester film. Additionally, flex circuits can be screen printed silver circuits on polyester. These flexible printed circuits (FPCs) are typically made by photolithography. An alternative way of making flexible foil circuits is laminating very thin (e.g. 0.07 mm) copper strips in between two layers of PET. These PET layers, typically 0.05 mm thick, are coated with an adhesive which is thermosetting, and will be activated during the lamination process. These techniques may be used in the production of the present plasma cell. It will be noted however that the electrode arrangement of the present plasma cell is designed to carry high voltages (e.g. 1 to 3 kV) and high frequencies (e.g. above 100 kHz), whereas known flexible circuit boards are designed to carry low potentials at low frequencies.

The flexibility of the dielectric substrate means that it can be shaped to correspond with a former inside the device. The former may for example be a quartz tube or part of the nozzle attachment. This substrate flexibility allows more scope for positioning the plasma cell within the device leading to more efficient use of space and contributing to a reduction in size of the device or if preferred to an allowable increase of size of other components within the device such as the power source.

In the example shown in Figure 3, two electrodes are shown and are connected to the source of electrical power by electrical connectors 52, 54. The connectors and the electrode patterns can be seen most clearly in Figure 3d, in which other components of the cell have been removed. The pattern is configured to enhance the generation of active species in the cell and may consist of any suitable shapes such as coils, zigzags or curvilinear tracks. Printing the pattern enables complex and suitable patterns to be produced without significant expense and without the risk of short-circuiting between tracks. Preferably the pattern covers as much of the surface of the cell as possible so that a generally uniform electric field is applied to gas in the cell. The patterns may be formed without abrupt corners or sharp points since it will be appreciated that such regions may attract a relatively high number of charge carriers which in turn may produce a non-uniform electric field.

The generally cylindrical plasma cell 40 may have an outside diameter of 3 to 10 mm and an exit nozzle diameter of 0.5 to 2mm. The dielectric substrate layers 46, 48 may be 0.1 to 1 mm thick. The electrode strands may be approximately 0.01 mm to 0.1 mm in width and thickness. The protective layer may be approximately 1 mm thick.

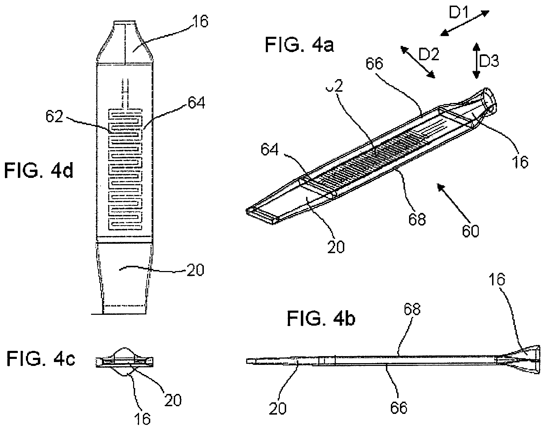

Whilst a generally cylindrical plasma cell is shown in Figure 3, other shapes may made from the flexible components, for example a cell which conveys gas along a tortuous path. Such an arrangement increases the residence time of gas in the cell and promotes plasma formation. Another plasma cell is shown in Figure 4 which has a flatter shape.

Referring to Figure 4, a plasma cell 60 is shown comprising electrodes 62, 64 printed on a dielectric substrate 66. Like reference numerals in Figure 4 will be used to denote like features discussed above and will not be explained again for brevity. A second dielectric layer 68 covers the electrode pattern, such that the electrode is embedded within the dielectric material. In this example, the dielectric substrate 66 is formed into a generally planar configuration. In this regard, the substrate has a substantially greater extent in a first dimension D1 extending between the inlet 16 and outlet 20 along the flow path and a second dimension D2 generally lateral to the first dimension than in a third dimension D3 generally orthogonal to said first and second dimensions. As shown the first dimension extends generally through the chamber, the second dimension extends across the chamber and the third dimension extends in the thickness of the chamber.

The benefits of the planar cell are threefold. Firstly, the gas is exposed to the electric field for a relatively long period as it passes through the chamber in the first dimension. Secondly, for each unit length in the first dimension, a relatively large amount of gas is exposed to the electric field because of the relatively large width in the second dimension. Thirdly, the relatively small thickness of chamber ensures that the maximum distance of any gas passing through the chamber is only a short distance from the or each electrode, whilst still allowing reasonable gas flow the chamber. It should also be noted that the internal surface area of the plasma chamber is large compared to the volume of gas and therefore is conducive to transporting heat away from the gas. In the example shown in Figure 1, the width of the chamber is about 10mm and the length is about 50mm. The height of the chamber is preferably less than 5 mm and more preferably less than about 2 mm.

In this example, the electrodes are transferred onto each planar side of the substrate 66 in a generally 'S' shape configuration. The electrodes cover only a portion of each planar side being spaced from its edges to reduce cross-over of the generated electric field around the edges rather than through the gas chamber in the cell.

The electrode pattern may not be continuous but may alternatively be provided in sections, or discrete patterns, which may be spaced apart one from another. The electrode(s) are preferably configured dependent on the particular characteristics of the cell, for example, the flow rate of gas through the cell, the half life of the active species generated in the cell and the type of treatment required.

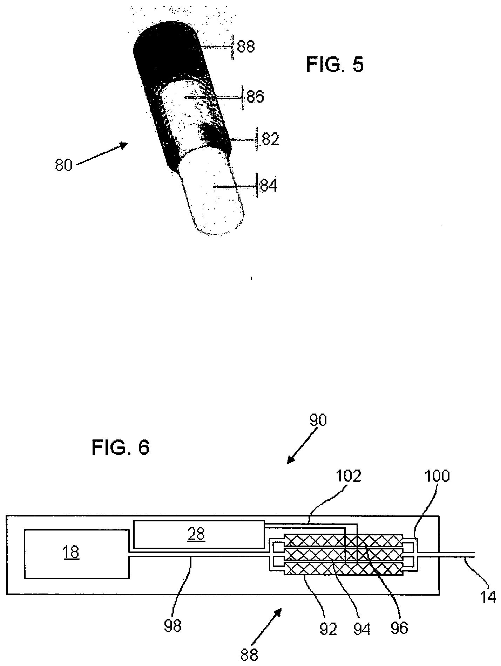

Another embodiment of the invention is shown in Figure 5. A plasma cell 80 is shown which comprises a generally tubular, or cylindrical, dielectric substrate 82 formed in this case from polyimide. A protective layer 84 which may be made of PTFE covers an inner surface of the dielectric substrate to resist degradation of the substrate during use. An electrode 86 is patterned onto the dielectric substrate. The electrode is made of a fibrous matrix which in this example is steel braid. The electrode pattern is a grid of fibres in this Figure but it will be appreciated that any suitable pattern may be formed. Simple experimentation, involving varying the voltage and frequency, will reveal which pattern performs well and establishes a good electric field in the plasma cell. The electrode pattern may be formed by first transferring a layer of steel, copper or other conductive material to the dielectric substrate and then using a laser to remove material to produce the desired pattern. Alternatively, the fibrous matrix may be transferred to the substrate during the extrusion process. A protective sheath 88 covers the electrode pattern and the dielectric substrate. The sheath provides mechanical support and electrical insulation. Polyimide may be used to form the sheath.

Microlumen® makes suitable tubular structures although for use in the field of medicine where the tubes are used as catheters. The steel braid which is transferred to the polyimide layer provides the tube with structural resilience and is not designed to carry electricity. The polyimide substrate provides a flexible material to allow ending when inserted in the body. It will be appreciated that the size of such tubes are necessarily small (about 1 to 3 mm) to fit inside bodily tracts and such a size also lends itself to use as a plasma cell for the reasons described in detail above.

Referring by way of example to Figure 2, the plasma cells described herein may be manufactured by patterning an electrode 26 on a dielectric substrate 22, configuring the dielectric substrate 22, for example into a cylinder, to form a flow path for gas from a cell inlet 16 to a cell outlet 20, and forming a protective lining 32 on an inner surface of the dielectric substrate for resisting reaction of the active species with the dielectric substrate. The order of the steps may be selected as required.

In examples shown in Figures 3 and 4, the patterned electrode is deposited on the dielectric substrate by printing techniques known in the manufacture of printed circuit boards. For example, in a subtractive process, a layer of copper may be bonded over the entire substrate, (creating a "blank PCB") then removing unwanted copper after applying a temporary mask (e.g. by etching), leaving only the desired copper traces. Alternatively, in an additive process, the conductive pathways may be made by depositing traces to the bare substrate (or a substrate with a very thin layer of copper) usually by a complex process of multiple electroplating steps.

The vast majority of circuit boards remain flat in use. However, in a preferred method of manufacturing a plasma cell the dielectric substrate is made from a thin film flexible dielectric material onto which the electrode is patterned. The substrate can then subsequently be shaped to enclose the flow path between the inlet and the outlet, for example as a cylinder, or in a form that that does not follow a straight path between the outlet and the inlet. Alternatively, the circuit can be inserted into a quartz or other dielectric material tube, where it will conform to the shape of the tube. In this way, the plasma cell can be manufactured by the relatively inexpensive printing of conductive tracts on a planar substrate and then formed into the required shape. The protective lining may be formed onto one surface of a planar substrate whilst the electrode pattern is printed on an opposing surface.

Referring to Figure 5, the patterned electrode is formed of a fibrous matrix which is transferred onto the dielectric substrate either during extrusion of the tubular substrate or subsequent to its manufacture. Since the material selected for the substrate is flexible, the plasma cell can subsequently be formed into any desired shape.

In the present embodiments, the selection of the dielectric material of the substrate should preferably take account of its thermal conductivity and in this regard, polyimide has a relatively good thermal conductivity of around 0.5 W/m.K, so that heat may be conducted away from the gas in the cell. The temperature of the gas mixture discharged from the plasma chamber is preferably less than 60°C, and more preferably less than 40°C.

The electrode(s) may be patterned generally uniformly on the dielectric substrate or may be patterned to produce one region which has a different concentration of conductive tracts than another region. For instance, it may be desirable to produce a stronger electric field towards the outlet of the cell compared towards the inlet of the cell, such that more energy is supplied to the gas as it approaches the treatment region. Alternatively, the electrode pattern may consist of multiple discrete patterns in series spaced apart along the flow path one from another.

The device of the embodiments having the plasma cells described herein lends itself to a compact form and in a preferred arrangement the device is configured to be hand-held and operated, for example, like an electric tooth brush may be hand-held and operated. A hand-held device must be sufficiently small and light that is not unwieldy in use and may be guided relatively precisely for application of generated active species to a treatment region such as a specific tooth in a mouth. In this regard, the device may be configured to have a mass of less than 1 kg, a length of less than 200mm and a width of 50mm.

A further device is shown in Figure 6. Since the plasma cells as described herein may be relatively small (e.g. 50mm length by 5 mm width), a plasma cell array 88 comprising a plurality of plasma cells may be provided in a single device, which may itself be suitable to be hand-held and operated. In Figure 6, the device 90 comprises three plasma cells 92, 94, 96 each of which are in flow communication with the source of gas 18 for receiving into the cells gas to be energised and with the nozzle 14 (or each nozzle) for plasma to be delivered from the cells to a treatment region. A gas duct 98 extends from the gas source and trifurcates to deliver gas to each of the cells. Further ducts 100 extend from the cell outlets and converge to deliver active species to the nozzle. The electrode(s) of each cell are connected by electrical conductors 102 to the source of electrical power 28.

The plasma cell array as shown is capable of delivering a greater amount of active species to the treatment region than the single plasma cell of the device shown in Figure 1. However, unlike a device that simply incorporates a larger plasma cell, the provision of the plasma cell array allows the gas to be in closer proximity to the electrodes of the cells and therefore interact more readily with the electric fields generated. In a larger cell, the maximum distance between the gas and the electrodes is increased and therefore a larger potential would have to be created at the electrode to deliver comparable energy to the gas.

Although in this example, the plasma cell array comprises three plasma cells, any number of cells may be incorporated. Further, the three plasma cells are disposed in parallel relation whereas one or more of the cells may be provided in series, however, a series relationship may be appropriate only if the half life of the active species is sufficiently long that plasma generated in the first of the series survives for application to the treatment region.

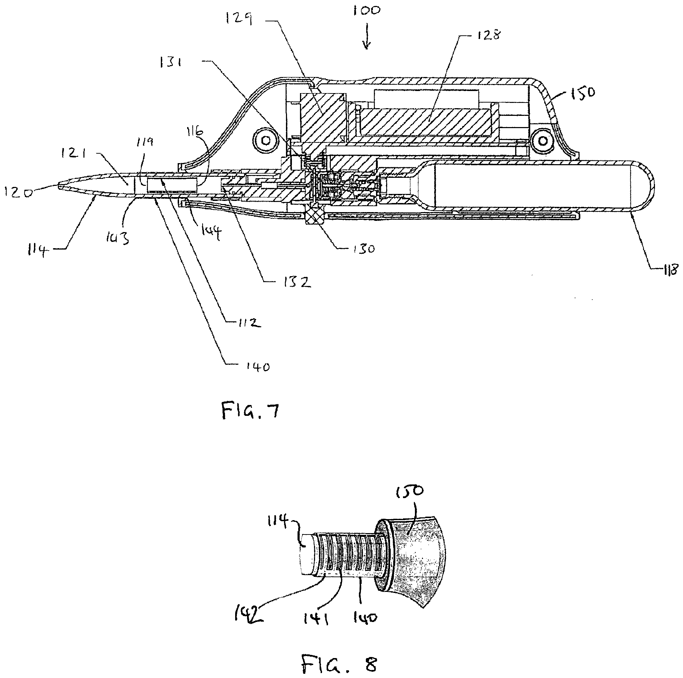

Figure 7 shows a further device 100 having an alternative arrangement. In a similar fashion to the device 10 of Figure 1, the device 100 comprises a plasma cell 112 for forming a gaseous plasma to be discharged through nozzle 114 (via nozzle outlet 120). The plasma cell 112 comprises an inlet 116 for receiving gas from a source 118 and an outlet 119 for discharging active species generated in the cell to a plasma chamber 121 located upstream (in use) of the nozzle outlet 120.

The plasma cell 112 has substantially the same configuration as the plasma cell shown in Figure 2. However, it will be understood that the plasma cell 112 may have any configuration consistent with any of the plasma cell configurations described hereinabove. Specifically, the electrode 26 may be of any other configuration than the spiral configuration described with reference to Figure 2, and may be embedded within, or sandwiched between, dielectric substrate 22. The electrode 26 of the plasma cell 112 is connected to the high voltage side of a high voltage transformer 129 which is powered by a lithium battery 128. In this way, gas travelling, in use, along a flow path from the inlet 116 to outlet 119 of plasma cell 112 is energised to form active species.

A housing 150 houses the components of the device 100 and the device further comprises a pressure regulator 131 and operating button 132. The operating button 132 may be operable to both open the gas supply to the plasma cell 112, and energise the high voltage transformer 129. Alternatively, separate switches may be provided for this purpose.

The nozzle 114 of the device 100 may be integral with the protective sheath 36 of the plasma cell 112. However, in the example shown in Figure 7, the nozzle 114 is separate from the plasma cell 112. The plasma cell 112 is fixed within the nozzle 114 adjacent the wall of the housing 150.

The device 100 further comprises an earth electrode 140. The earth electrode 140 is substantially cylindrical in shape and fixed to the outer surface of the nozzle 114 adjacent to the wall of the housing 150. As shown in Figure 7, the earth electrode 140 is concentric with the plasma cell 112 and overlaps the plasma cell such that the outermost end 143 of the earth electrode 140 is closer to the nozzle outlet 120 than the discharge end 119 of the plasma cell 112. Similarly, the innermost end 144 of the earth electrode 140 is closer to the nozzle outlet 120 than the inlet end 116 of the plasma cell 112. This arrangement serves to urge the plasma along the nozzle 114 towards the nozzle outlet 120. In one preferred embodiment, the earth electrode 140 may be slid axially in relation to the plasma cell 112 to allow for adjustment in the capacitance of the plasma cell 112 to optimise the plasma formation, provide more efficient power coupling to the power supply and vary the region of highest field intensity.

As shown in Figure 8, the earth electrode 140 comprises an electrode 141 (Figure 8) formed on a dielectric substrate 142. The dielectric substrate 142 may be made of any suitable material but is preferably made of a polyimide. The electrode 141 may alternatively be sandwiched between layers of polyimide substrate or embedded within it. Alternatively or additionally, an alternative outer layer material may be provided for the earth electrode 140, For example, the outer surface of the earth electrode 140 may be provided with a polyether block amide (or other suitable material) protective sheath in addition to, or in place of, the outermost polyimide layer. The electrode 142 is connected to the earth side of the high voltage transformer 129.

The illustrated arrangement of the earth electrode 140 relative to the plasma cell 112 has the effect of making the device 100 more efficient than it would be were the earth electrode 140 not present. This is because the electric field produced by the earth electrode 140 (when energised) shields the electric field produced by the plasma cell 112 (when energised). This has the effect that less energy is required to produce the desired level of active species in the plasma exiting the cell 112, thereby making the device 100 more efficient. The effect of the earth electrode 140 is to increase the field intensity and to act to control the near field effect of conductive objects influencing the plasma jet detrimentally. The earth electrode 140 provides control over the earthing by virtue of the dielectric value of the substrate 142 and the substrate 22 of the encapsulated plasma cell 112, in effect controlling the capacitance.

The entire nozzle arrangement consisting of nozzle 114, plasma cell 112 and earth electrode 140 may be a monolithic assembly that can be removed from the device 100. Preferably the nozzle assembly is disposable so that it may readily be removed and replaced. This is beneficial for reasons of hygiene. In this case the necessary electrical connections are made via plugs which connect to the inner and outer electrodes when a new nozzle assembly is fitted. The invention is set out in the following claims:

## Claims

1. A device (10) for forming at an ambient atmospheric pressure a gaseous plasma comprising active species for treatment of a treatment region, the device (10) comprising:
at least one plasma cell (12) for forming said gaseous plasma for treating the treatment region, the at least one plasma cell (12) comprising:
an inlet (16) for receiving gas from a source and an outlet (20) for discharging active species generated in the cell;
a dielectric substrate (22) enclosed around a flow path (24) for gas conveyed from the inlet (16) to the outlet (20);
an electrode (26) formed on or in the dielectric substrate (22) for energising gas along the flow path (24) to form active species; and
a protective coating (32) made of a dielectric formed on an inner surface of the dielectric substrate (22) for protecting the dielectric substrate (22) from reaction with the active species,
the device (10) further comprising:
a nozzle (114) having a nozzle outlet (120) and a plasma chamber (121) located upstream of the nozzle outlet (120); wherein the plasma cell (12, 112) is fixed within the nozzle (114), and wherein the outlet of the plasma cell is configured to discharge active species generated in the plasma cell (12, 112) to the plasma chamber (121); and an earth electrode (140) comprising a dielectric substrate (142) and an electrode (141) formed on or in the dielectric substrate (142), wherein the earth electrode (140) substantially surrounds and at least partially overlaps the at least one plasma cell (12), wherein the outermost end of the earth electrode (140) is closer to the nozzle outlet (120) than the outlet (20) of the plasma cell (12).

2. A device (10) according to claim 1, wherein the dielectric substrate (22 and/or 142) of the at least one plasma cell (12) and/or the earth electrode (140) is made of a polyimide.

3. A device (10) according to claim 1 or 2, wherein the protective coating (32) is made of a material selected from one of PTFE, FEP or silicone rubber being generally un-reactive with the active species.

4. A device (10) according to any preceding claim, wherein the electrode (26 and/or 141) of the at least one plasma cell (12) and/or the earth electrode (140) is formed by patterning an electrically conductive material on the respective dielectric substrate (22).

5. A device (10) according to claim 4, wherein the electrode (26 and/or 141) is printed.

6. A device (10) according to claim 4, wherein the patterned electrode (26 and/or 141) is formed of a fibrous matrix transferred onto the respective dielectric substrate (22 and/or 142).

7. A device (10) according to anyone of the preceding claims, wherein the dielectric substrate (22) of the at least one plasma cell (12) is flexible and is shaped to define the flow path (24).

8. A device (10) according to claim 7, wherein the dielectric substrate (22) of the at least one plasma cell (12) is formed by a flexible tube enclosing the flow path (24).

9. A device (10) according to any of the preceding claims, comprising a protective sheath (34) formed around the dielectric substrate (22) and electrode (26) of the at least one plasma cell (12).

10. A device (10) according to any of the preceding claims comprising a plasma cell (88) array having a plurality of said plasma cells.

## Patentansprüche

1. Vorrichtung (10) zum Ausbilden, bei einem Umgebungsatmosphärendruck, eines gasförmigen Plasmas, umfassend aktive Spezies für die Behandlung eines Behandlungsgebiets, wobei die Vorrichtung (10) Folgendes umfasst:
mindestens eine Plasmazelle (12) zum Ausbilden des gasförmigen Plasmas zum Behandeln des Behandlungsgebiets, wobei die mindestens eine Plasmazelle (12) Folgendes umfasst:
einen Einlass (16) zum Empfangen von Gas von einer Quelle und einen Auslass (20) zum Austragen von in der Zelle generierten aktiven Spezies;
ein dielektrisches Substrat (22), das um einen Strömungsweg (24) für Gas eingeschlossen ist, das vom Einlass (16) zum Auslass (20) befördert wird;
eine Elektrode (26), die auf oder in dem dielektrischen Substrat (22) ausgebildet ist, zum Erregen von Gas entlang dem Strömungsweg (24), um aktive Spezies auszubilden; und
eine Schutzbeschichtung (32), die aus einem Dielektrikum besteht, auf einer inneren Oberfläche des dielektrischen Substrats (22) ausgebildet zum Schützen des dielektrischen Substrats (22) vor
einer Reaktion mit den aktiven Spezies,
wobei die Vorrichtung (20) weiterhin Folgendes umfasst:
eine Düse (114) mit einem Düsenauslass (120) und einer Plasmakammer (121), die sich vor dem Düsenauslass (120) befindet; wobei die Plasmazelle (12, 112) innerhalb der Düse (114) fixiert ist und wobei der Auslass der Plasmazelle konfiguriert ist zum Austragen von in der Plasmazelle (12, 112) generierten aktiven Spezies zur Plasmakammer (121); und
eine Erdelektrode (140), die ein dielektrisches Substrat (142) und eine Elektrode (141), die auf oder in dem dielektrischen Substrat (142) ausgebildet ist, umfasst, wobei die Erdelektrode (140) die mindestens eine Plasmazelle (12) im Wesentlichen umgibt und zumindest teilweise überlappt,
wobei das äußerste Ende der Erdelektrode (140) sich näher an dem Düsenauslass (120) als an dem Auslass (20) der Plasmazelle (12) befindet.

2. Vorrichtung (10) nach Anspruch 1, wobei das dielektrische Substrat (22 und/oder 142) der mindestens einen Plasmazelle (12) und/oder die Erdelektrode (140) aus einem Polyimid bestehen.

3. Vorrichtung (10) nach Anspruch 1 oder 2, wobei die Schutzbeschichtung (32) aus einem Material besteht, das ausgewählt ist aus einem von PTFE, FEP oder Silikonkautschuk, die im Allgemeinen mit den aktiven Spezies nicht reagieren.

4. Vorrichtung (10) nach einem vorhergehenden Anspruch, wobei die Elektrode (26 und/oder 141) der mindestens einen Plasmazelle (12) und/oder die Erdelektrode (140) ausgebildet wird durch Strukturieren eines elektrisch leitenden Materials auf dem jeweiligen dielektrischen Substrat (22).

5. Vorrichtung (10) nach Anspruch 4, wobei die Elektrode (26 und/oder 141) gedruckt ist.

6. Vorrichtung (10) nach Anspruch 4, wobei die strukturierte Elektrode (26 und/oder 141) aus einer auf das jeweilige dielektrische Substrat (22 und/oder 142) übertragenen Fasermatrix ausgebildet ist.

7. Vorrichtung (10) nach einem der vorhergehenden Ansprüche, wobei das dielektrische Substrat (22) der mindestens einen Plasmazelle (12) flexibel ist und geformt ist zum Definieren des Strömungswegs (24).

8. Vorrichtung (10) nach Anspruch 7, wobei das dielektrische Substrat (22) der mindestens einen Plasmazelle (12) durch einen den Strömungsweg (24) einschließenden Schlauch ausgebildet ist.

9. Vorrichtung (10) nach einem der vorhergehenden Ansprüche, umfassend einen Schutzmantel (34), der um das dielektrische Substrat (22) und die Elektrode (26) der mindestens einen Plasmazelle (12) herum ausgebildet ist.

10. Vorrichtung (10) nach einem der vorhergehenden Ansprüche, umfassend ein Plasmazellen-(88)-Array mit mehreren der Plasmazellen.

## Revendications

1. Dispositif (10) destiné à former à une pression atmosphérique ambiante un plasma gazeux comprenant des espèces actives pour le traitement d'une région de traitement, le dispositif (10) comprenant :
au moins une cellule à plasma (12) destinée à former ledit plasma gazeux pour traiter la région de traitement, l'au moins une cellule à plasma (12) comprenant :
une entrée (16) destinée à recevoir du gaz provenant d'une source et une sortie (20) destinée à évacuer des espèces actives générées dans la cellule ;
un substrat diélectrique (22) refermé autour d'un passage (24) pour du gaz transporté de l'entrée (16) à la sortie (20) ;
une électrode (26) formée sur ou dans le substrat diélectrique (22) destinée à exciter le gaz le long du passage (24) pour former des espèces actives ; et
un revêtement protecteur (32) constitué d'un diélectrique formé sur une surface intérieure du substrat diélectrique (22) pour protéger le substrat diélectrique (22) d'une réaction avec les espèces actives,
le dispositif (10) comprenant en outre :
une buse (114) ayant une sortie de buse (120) et une chambre à plasma (121) située en amont de la sortie de buse (120) ; la cellule à plasma (12, 112) étant fixée à l'intérieur de la buse (114), et la sortie de la cellule à plasma étant configurée pour évacuer des espèces actives générées dans la cellule à plasma (12, 112) dans la chambre à plasma (121) ; et
une électrode de terre (140) comprenant un substrat diélectrique (142) et une électrode (141) formée sur ou dans le substrat diélectrique (142), l'électrode de terre (140) entourant sensiblement et chevauchant au moins partiellement l'au moins une cellule à plasma (12),
dans lequel l'extrémité extérieure de l'électrode de terre (140) est plus proche de la sortie de buse (120) que la sortie (20) de la cellule à plasma (12).

2. Dispositif (10) selon la revendication 1, dans lequel le substrat diélectrique (22 et/ou 142) de l'au moins une cellule à plasma (12) et/ou de l'électrode de terre (140) est constitué d'un polyimide.

3. Dispositif (10) selon la revendication 1 ou 2, dans lequel le revêtement protecteur (32) est constitué d'un matériau choisi à partir d'un élément parmi le PTFE, le FEP ou le caoutchouc de silicone qui est généralement inerte vis-à-vis des espèces actives.

4. Dispositif (10) selon une quelconque revendication précédente, dans lequel l'électrode (26 et/ou 141) de l'au moins une cellule à plasma (12) et/ou de l'électrode de terre (140) est formée en modelant un matériau électriquement conducteur sur le substrat diélectrique respectif (22).

5. Dispositif (10) selon la revendication 4, dans lequel l'électrode (26 et/ou 141) est imprimée.

6. Dispositif (10) selon la revendication 4, dans lequel l'électrode modelée (26 et/ou 141) est constituée d'une matrice fibreuse transférée sur le substrat diélectrique respectif (22 et/ou 142).

7. Dispositif (10) selon l'une quelconque des revendications précédentes, dans lequel le substrat diélectrique (22) de l'au moins une cellule à plasma (12) est souple et est façonné pour définir le passage (24).

8. Dispositif (10) selon la revendication 7, dans lequel le substrat diélectrique (22) de l'au moins une cellule à plasma (12) est formé par un tube souple délimitant le passage (24).

9. Dispositif (10) selon l'une quelconque des revendications précédentes, comprenant une gaine protectrice (34) formée autour du substrat diélectrique (22) et de l'électrode (26) de l'au moins une cellule à plasma (12).

10. Dispositif (10) selon l'une quelconque des revendications précédentes comprenant un réseau de cellules à plasma (88) ayant une pluralité desdites cellules à plasma.
